# EUROPEAN PATENT SPECIFICATION

(11) **EP 3 411 355 B1**
(45) Date of publication and mention of the grant of the patent: **28.10.2020**
(21) Application number: 17704191.0
(22) Date of filing: 03.02.2017
(51) Int. Cl.: C07C 227/32, C07C 229/48

(54) **PROCESS FOR THE PREPARATION OF TRANS-4-AMINO-1-CYCLOHEXANECARBOXYLIC ACID**
VERFAHREN ZUR HERSTELLUNG VON TRANS-4-AMINO-1-CYCLOHEXANCARBONSÄURE
PROCÉDÉ DE PRÉPARATION DE L'ACIDE TRANS-4-AMINO-1-CYCLOHEXANE CARBOXYLIQUE

(30) Priority: 05.02.2016 EP 16154408
(43) Date of publication of application: 12.12.2018
(73) Proprietor: Siegfried AG, 4800 Zofingen (CH)
(72) Inventor: WEBER, Beat, 4800 Zofingen (CH); JACEK, Olma, 42-600 Tarnowskie Góry (PL); BRYSZ, Michal, 32-065 Krzeszowice (PL); LUKASZEWICZ, Ewa, 94-218 Lódz (PL)
(74) Representative: Harms, Guido
(86) International application number: PCT/EP2017/052361
(87) International publication number: WO 2017/134212

(56) References cited:
- EP-B1- 2 384 326
- IN-A- 1776M UM2 008
- S. ÖBÖLÖS, ET AL.: "Highly selective preparation of trans-4-aminocyclohexane carboxylic acid from cis-isomer over Raney nickel catalyst" In: S.R. SCHMIDT: "Catalysis of Organic Reactions: Twenty-first Conference", 7 December 2006 (2006-12-07), CRC Press, Boca Raton, Florida, USA, XP008180865, ISBN: 978-0-8493-7557-6 pages 45-53, cited in the application table 1, entries 5-8

## Description

The present invention relates to a process for the preparation of trans-4-amino-1-cyclohexanecarboxilic acid derivatives (also the "trans-product", "trans-isomer" or the "trans derivative") and its use in the preparation of active pharmaceutical ingredients.

### State of the art

Trans-4-amino-1-cyclohexanecarboxylic acid derivatives are useful intermediates for the synthesis of optically active chemical compounds. For example, EP2384326 reports the use of trans-4-(t-butyloxycarbonyl)amino-1-cyclohexanecarboxylic acid for the synthesis of Janus Kinase inhibitors. It is also used in the synthesis of other useful active pharmaceutical ingredients, see WO9825897. The words trans or cis in this invention relate to the relative stereochemistry of the nitrogen and the carboxylic acid moiety at the cyclohexane ring.

Furthermore trans-4-amino-1-cyclohexanecarboxylic acid derivatives have been shown to be good substituents to daunorubicin and adriamycin. (Synthesis of N-Substituted Amino Cyclohexanecarboxylic Acid esters of Daunomycin and Ariamycinon, Li Jiebing,and Zhang Chunnian (Shanghai Institute of Pharmaceutical Industry)

This might be because of its ability to serve as dynorphin A(1-13)NH₂ analog (Synthesis and opioid activity of dynorphin A-(1-13)NH₂ analogs containing cis- and trans-4-aminocyclohexanecarboxylic acid, Journal of Medicinal Chemistry, 1993; 36(8):1100-1103)

It is well known that 4-Amino-1-cyclohexanecarboxilic acid can be prepared by the hydrolytic reduction of 4-nitro-benzoic acid via the 4-Amino-benzoic acid intermediate.

The synthesis of the trans-isomer, however has ever been a challenge, since the synthesis from benzene derivatives always yield in the cis-isomer as described in FR1473246:

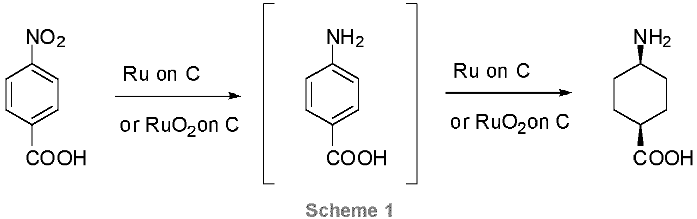

The state of the art reports a yield of 83 % with 99,8 % cis configuration.

Whenever a ratio of trans and cis isomers is given, the ratio is expressed in amount trans to amount cis or in percentage of trans isomer compared to the mixture. For example a ratio of trans to cis of 4/1 or 4:1 is equal to 80 % trans-product in the reaction mixture. The ratios are determined by NMR data.

Maegawa et al report the Hydrogenation of Aminobenzoic acid under mild conditions (1.01 MPa (10 atm), iPrOH, 60 °C) with Rhodium on Carbon catalyst, but they do not report the cis/trans ratio of the reaction. (Maegawa et al. "Efficient and Practical Arene Hydrogenation by Heterogeneous Catalysts under Mild Conditions" Chemistry - A European Journal (2009), 15(28), pp. 6953-6963)

Palaima et al report the catalytic hydrogenation of Aminobenzoic acid to the corresponding trans-4-amino-1-cyclohexanecarboxylic acid with Raney Nickel. (Palima A.l. et al.: "cis-3- and trans-4-aminocyclohexanecarboxylic acids and their esters." Bulletin of the academy of sciences of the USSR. Division of chemical science, vol. 26, no. 1, 20 July 1977, pages 171-172.)

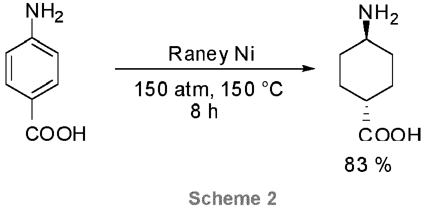

However, the use of Raney Ni, although being a rather cheap catalyst, has some disadvantages, as there is firstly the problem in correct dosing the suspended material, secondly the pyrophorous behavior if not handled in water and as a third, the difficulty in separation of the product from residues of fine dispersed Nickel particles in the reaction mixture. Furthermore, the reaction with Raney Nickel is conducted at a pressure of around 150 bar, which is no issue on laboratory scale, but limits the use of the reaction in industrial scale, because only small volume reactors may be loaded and the reaction is not suitable for multipurpose plants.

Further researchers provide methods for the isomerization of the cis isomer into the trans isomer by means of forming a derivative with bulky substituents at the amine and then subsequently treat this derivative with base in methanol to convert the cis isomer into the trans isomer.

The following scheme shows this approach as described in EP1249233.

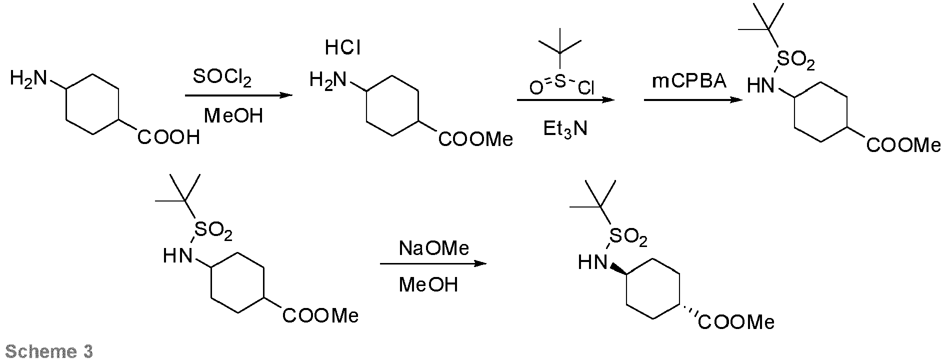

The overall yield of this transformation is reported with 40 %. This makes this synthesis less attractive for industrial production.

EP1484301 describes the conversion of cis 4-amino-1-cyclohexanecarboxylic acid via two alternative intermediates (Path 1 and path 2) to the trans configurated 4-Amino-1-cyclohexanecarboxylic acid in an improved process as described below:

### Path 1

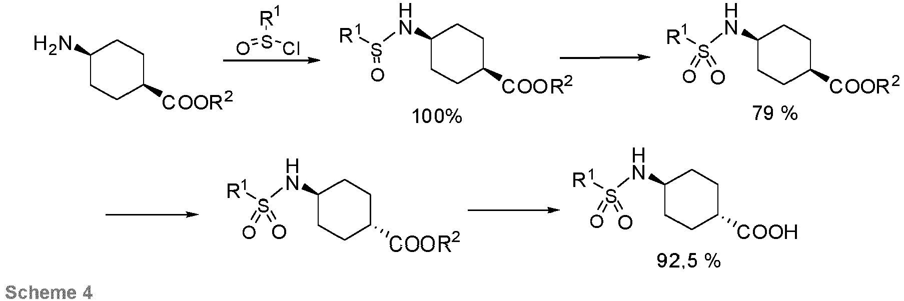

wherein R¹ and R² are each independently a straight or branched C1 to C6 alkyl.

The process involves the use of a base in an aprotic solvent for the conversion from cis to trans and the crystallization of the final compound in an aprotic solvent.

The yield of the conversion (including the conversion to the sulfonate) is reported to be 73 %.

### Path 2:

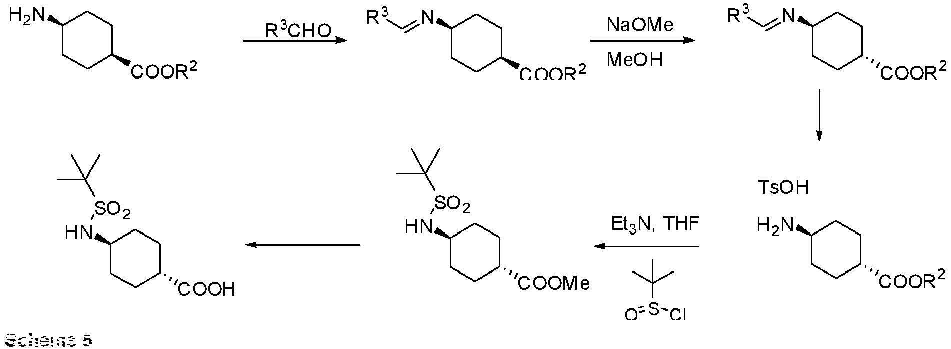

wherein R2 is a straight or branched C1 to C6 alkyl and R3 is optionally substituted phenyl.

The overall yield is reported to be 68 %. The process involves the use of a base in an organic solvent for the conversion from cis to trans.

Further inventions describe the epimerization of the cis 4-amino-1-cyclohexanecarboxylic acid to the trans derivative. Either as free base (US4,816,484) or N-protected (IN1776mum2008, US7,314,950)

These numerous inventions of the state of the art describing the conversion of cis to trans derivatives emphasize the need of an industrially applicable direct synthesis of the trans configurated derivatives under mild conditions.

Göbölös et al have reported that it is possible to convert p-aminobenzoic acid to 4-amino-1-cyclohexanecarboxylic acid with a cis/trans ratio of 1:1 when using Ru on Al₂O₃ or Rh on Al₂O₃ support. (Schmidt, Stephen R., ed. Catalysis of Organic Reactions: Twenty-first Conference. CRC Press, 2006. Highly Selective Preparation of trans-4-Aminocyclohexane Carboxylic Acid from cis-Isomer over Raney® Nickel Catalyst Sándor Gőbölös, Zoltán Banka, Zoltán Tóth, János Szammer, and József L. Margitfalvi)

The authors also show the direct conversion of the cis-isomer of 4-Amino-1-cyclohexanecarboxylic acid to the trans isomer with Raney Nickel. It could be achieved a trans isomer content of 70 % which corresponds to a cis/trans ratio of 1:2.3. For the disadvantages of using Raney Nickel see above.

The problem to be solved with this invention is thus, to provide a method to convert p-Aminobenzoic acid and its derivatives to the corresponding 4-Amino-1-cyclohexanecarboxylic acid or its derivative with a high trans:cis ratio under mild conditions.

The content of trans-product is preferably more than 75 %.

Description of the invention.

We have now found that the direct conversion of p-Aminobenzoic acid to the desired 4-amino-1-cyclohexanecarboxylic acid in trans form can be achieved with a trans product ratio of more than 75 %. The result is achieved in one step (one pot) without the need of conversion of the cis-isomer into the trans-isomer.

The invention shows a one-pot process for the preparation of a compound of the formula I with a trans ratio of more than 75 %. 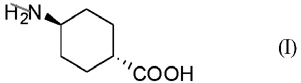 comprising reacting a compound of the formula II

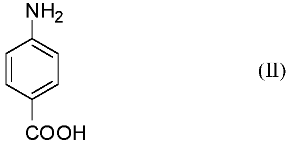

The reaction is conducted in one step. One step in this context means that for achieving the desired result it is not necessary to change the solvent or reactants in the reaction mixture nor is it necessary to use more than one vessel. The product may be isolated or the reaction mixture may be used without further purification for the derivatization of the reaction product.

According to the invention the reaction is conducted under basic conditions using a catalyst containing Ruthenium. The support may be carbon.

If using Ruthenium as a catalyst, the yield of the reaction tends to be higher. Furthermore, Ruthenium is cheaper than Rhodium. Therefore Ruthenium is a preferred catalyst either used as neat metal or as its oxide.

The reaction is conducted in the presence of an appropriate solvent or solvent mixture. The solvents may be selected from Water, Acetone, isopropanol, ethanol or methanol.

Best results have been achieved with a mixture of water and isopropanol, whereas also water would be a feasible solvent alone.

The reaction gives lower isomer ratios when the temperature is 85 °C or below, or 138 °C and higher. The preferred temperature range is between 90 °C and 120 °C. More preferred is a temperature range of 100 - 115 °C. Most preferred Temperature for the reaction is approximately 100 °C.

It has now surprisingly been found that the reaction may be conducted under very mild conditions.

Using a hydrogen pressure that is below 100 bar opens the possibility to perform bigger batch sizes and also opens the possibility to perform reactions in multi-purpose vessels. This circumstance is even more distinct as the pressure used in the reaction decreases.

The reaction is performed at a pressure of not more than 15 bar, most preferred not more than 10 bar.

The most versatile and therefore most widely usable molecule to be produced is the unsubstituted 4-amino-1-cyclohexanecarboxylic acid (Compound (I)). Therefore it is preferred to prepare with the above described invention the 4-amino-1-cyclohexanecarboxylic acid with a trans ratio of more than 75 % in one step.

The catalyst may be used in ratios between 1 and 100 %, calculated in weight/weight on the used starting material (compound (II)), to get satisfactory results. However, best results have been received at catalyst concentrations of 15-60 %. Preferred concentration of the catalyst is between 20 and 40 %. In this context, the term catalyst is to be understood as the metal and the solid support. The support may be loaded with different amounts of the metal or metal oxide. The usual amount of metal is between 5 and 10 % metal on the support. The best results in terms of trans/cis selection were obtained using 50 % of 5 % Ru/C.

Compound (I) may be further used in the synthesis of pharmaceutically active compounds.

Therefore it may be necessary to protect the amino group of compound (I). This may be performed with either of the following protecting groups:
tert-butyloxycarbonyl, 9-fluorenylmethyloxycarbonyl, acetyl, carbamate, p-methoxyphenyl, tosyl or other sulfonamides (nosyl & nps) groups.

The separation of the cis/trans mixture of compound (I) is not part of the present invention but may be achieved by various known methods like selective crystallization or selective esterification of the carbonic acid group.

Under conditions as described in example 3 it is possible to selectively react only the cis-4-amino-1-cyclohexanecarboxylic acid derivative (the cis-product) to the respective ester and to isolate pure trans product.

In one embodiment of the invention the preparation of trans-4-amino-1-cyclohexanecarboxylic acid is achieved by the reaction of p-aminobenzoic acid in an aqueous solution of 10 % NaOH with 25 % of 5 % Ru/C under Hydrogen pressure of 15 bar at 100 °C.

The mixture is then BOC-protected with 1 equivalent of BOC-anhydride without isolation of the reaction mixture of step 1 and subsequent esterification of the cis-product to give a total overall yield of 47 % from (II).

Although this invention only describes the synthesis starting from an amine substituted benzylcarbonic acid, it is obvious to the person skilled in the art, that also nitro-substituted derivatives will undergo the same reaction as the nitro-group will be reduced to the amine under the reaction conditions. See also Scheme 1.

Compound (I) may be used for the synthesis of a pharmaceutically active compound. It can, for example be used for the preparation of Oclacitinib.

It is possible to reuse the catalyst in another conversion, but the yield and isomer ratio is dependent on the work up and purification method of the catalyst.

### Examples

### Example 1

### Preparation of 4-aminocyclohexane-1-carboxylic acid (cis/trans mixture) (1):

p-Aminobenzoic acid (10.0 g, 0.07mol, 1eq.), 5 % Ru/C (2,50 g) and 10% NaOH (100.0mL) were mixed in autoclave. The mixture was stirred at 100°C under 15 bar of hydrogen. After 20h of stirring no Starting material was observed on TLC (DCM/MeOH/NH₃ = 5/5/1, v/v/v, stain: ninhydrine). According to the NMR - full conversion and cis:trans ratio = 1:4.6. Reaction was stopped.

The following table gives an overview about the performed examples according to the procedure as described in example 1.

| **Experiment performed** | | **Results** |
|---|---|---|
| **No.** | **Conditions** | **¹H NMR** |
| 1.1 | 10.00 g of p-aminobenzoic acid, 5.00 g 5%Ru/Al₂O₃, 10%NaOH in H₂O 10 vol. 100°C, 15 bar, 28h | Cis/rans 1/4.2 |
| 1.2 | 10.00 g of p-aminobenzoic acid and 5.00 g 5%Ru/C, 10%NaOH in H₂O 10 vol. 100°C, 15 bar, 20 h | Cis/trans 1/4 |
| 1.3 | 10.00 g of p-aminobenzoic acid, 2.50 g 5%Ru/C, 10%NaOH in H₂O 10 vol. 100°C, 15 bar, 20 h | Cis/trans 1/4.6 |
| 1.4 | 10.00 g of p-aminobenzoic acid, 5.00 g 5%Ru/C, 10%NaOH in H₂O 10 vol. 100°C, 15 bar, 20h | Cis/trans 1/4.3 |
| 1.5 | 10.00 g of p-aminobenzoic acid, 5.00 g 5%Ru/Al₂O₃, 10%NaOH in H₂O 10 vol. 100°C, 15 bar, 28h | Cis/rans 1/4.2 |

### Example 2 (not according to the invention)

### Preparation of 4-{[(tert-butoxy)carbonyl]amino}cyclohexane-1-carboxylic acid (cis/trans mixture) (2)

Catalyst from example 1 was not filtered. Acetone (327,3 mL, 30vol.) and Boc anhydride (16.63 g, 0.07 mol, 1.0 eq.) were added. Reaction mixture was stirred over 20h at room temperature. After that time TLC control (DCM/MeOH/NH₃ = 5/5/1, v/v/v, elicited in KMnO₄ (product sensitive) and in ninhydryne (substrate sensitive) showed only product, so reaction was finished. Catalyst was filtered through celite and washed with 600 mL of mixture: acetone/water = 4/1 ; v/v. Acetone was evaporated under reduced pressure. Water layer (pH =9) was extracted 3 times with Dichloromethane (DCM) (3x75mL). Water solution was acidified with 20 g of citric acid to pH=4 and Extracted 5 times with DCM (5x100mL). Combined organic layers were dried over Na₂SO₄, then filtered off and the organic solution was evaporated and dried overnight under reduced pressure to give 12,99 g, Yield: 70 % Purity: 92 %.

### Example 3 (not according to the invention)

### Separation of trans-4-{[(tert-butoxy)carbonyl]amino}cyclohexane-1-carboxylic acid from the cis-derivative

BOC-aminoacid ((2) 12.99 g, 0.05mol, 1.0eq, cis:trans=1:3.6) was mixed with K₂CO₃ (2 g, 0.06 mol, 1.2 eq with respect to the cis content in (2)) and suspended in acetone (259 mL, 20.0 vol.). To the reaction mixture bromoethane (1.93 mL, 2.82 g, 0.02 mol, 0.43eq - which was 2.0 eq calculated for cis isomer content in the mixture) was added under stirring. Reaction mixture was stirred at 60°C over 3h. White precipitate appeared. Reaction mixture was cooled to room temperature over 30 minutes and then stirred over 1hr at -10°C. After that time precipitate was filtered and washed with 100 mL of acetone (cooled to -10°C). Precipitate was added to 200 mL of 20% citric acid and 100 ml of DCM. Layers were separated. Water layer was extracted 4 times with DCM (4x100mL). Combined organic layers were dried over Na₂SO₄, then filtered off and solution was evaporated and dried under reduced pressure overnight to give trans-4-{[(tert-butoxy)carbonyl]amino}cyclohexane-1-carboxylic acid, 8.06 g (33mmol), purity 99.1 %, Yield 62 %.

## Claims

1. A one pot process for the preparation of a compound of formula I with a trans ratio of more than 75 %. 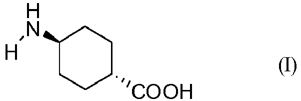 , comprising reacting a compound of the formula II 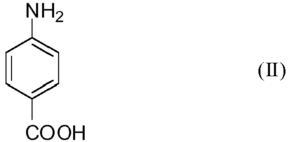 comprising the following reaction conditions:
- use of Ruthenium on Carbon as Catalyst
- use of a solvent selected from Water, Acetone, isopropanol, Ethanol, Methanol or mixtures thereof.
- reaction under basic conditions
- a reaction temperature of more than 90°C and less than 120 °C using hydrogen pressure of not more than 15bar.

2. Process according to claim 1 wherein the metal catalyst is used in 1-50 % of the used compound (II) weight/weight

3. Process according to claim 1 wherein the Hydrogen pressure is not more than 10 bar.

## Patentansprüche

1. Ein-Topf-Verfahren zur Herstellung einer Verbindung der Formel I mit einem Trans-Verhältnis von mehr als 75 %: 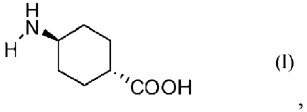 umfassend ein Reagieren einer Verbindung der Formel II: 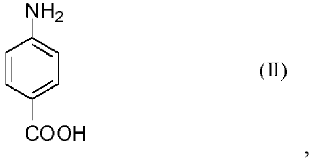 umfassend die folgenden Reaktionsbedingungen:
- Verwendung von Ruthenium-auf-Kohlenstoff als Katalysator,
- Verwendung eines Lösungsmittels, das aus Wasser, Aceton, Isopropanol, Ethanol, Methanol oder Gemischen davon ausgewählt ist,
- Reaktion unter basischen Bedingungen,
- eine Reaktionstemperatur von mehr als 90 °C und weniger als 120 °C,
unter Verwendung eines Wasserstoffdrucks von nicht mehr als 15 bar.

2. Verfahren nach Anspruch 1, wobei der Metallkatalysator in 1-50 Gew.-% der verwendeten Verbindung (II) verwendet wird.

3. Verfahren nach Anspruch 1, wobei der Wasserstoffdruck nicht mehr als 10 bar beträgt.

## Revendications

1. Procédé monotope destiné à la préparation d'un composé de formule I ayant un rapport trans supérieur à 75%, 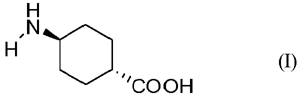 , comprenant la réaction d'un composé de formule II 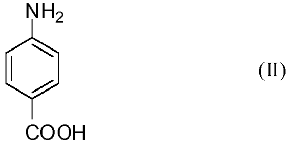 comprenant les conditions de réaction suivantes :
- utilisation de ruthénium sur carbone en tant que catalyseur
- utilisation d'un solvant choisi parmi l'eau, l'acétone, l'isopropanol, l'éthanol, le méthanol ou les mélanges de ceux-ci,
- réaction dans des conditions basiques
- une température de réaction supérieure à 90 °C et inférieure à 120 °C en utilisant une pression d'hydrogène inférieure à 15 bar.

2. Procédé selon la revendication 1, dans lequel le catalyseur métallique est utilisé dans 1 à 50 % du composé utilisé (II) en poids/poids.

3. Procédé selon la revendication 1, dans lequel la pression d'hydrogène est inférieure à 10 bar.
